# EUROPEAN PATENT APPLICATION

(11) **EP 4 623 846 A1**
(43) Date of publication of application: **01.10.2025**
(21) Application number: 24166858.1
(22) Date of filing: 27.03.2024
(51) Int. Cl.: A61B 18/08, A61B 5/00, A61B 18/14

(54) **METHOD FOR DETERMINING AN ABLATION PARAMETER FOR AN ABLATION TREATMENT**

(71) Applicant: Siemens Healthineers AG, 91301 Forchheim (DE)
(72) Inventor: Joy, Vaisakh Nappady, 91052 Erlangen (DE); Kratzke, Lisa, 91054 Erlangen (DE); Schäfer, Sebastian, 96146 Altendorf (DE); Boivin, Gael Luis, 8032 Zurich (CH)
(74) Representative: Siemens Healthineers Patent Attorneys

(57) **Abstract**

The invention relates to a method for determining an ablation parameter for an ablation treatment, comprising:
- Receiving (100) sensor data acquired by at least two sensors (10), said sensors (10) being spatially arranged along a shaft (5) of an ablation probe (2), each sensor having a distinct sensor position (11, T1, T2, T3, T4), wherein the sensor data for at least one measurement parameter includes two measured values obtained at different sensor positions (11, T1, T2, T3, T4);
- Determining (200) the ablation parameter based on the sensor data and/or the measured values of the same measurement parameter at different sensor positions (11, T1, T2, T3, T4);
- Providing (300) the determined ablation parameter.

## Description

Ablation therapy refers to a medical procedure where tumors or abnormal tissue are removed or destroyed using various techniques. The goal of ablation therapy is to eliminate or reduce the size of the tumor, thereby treating or alleviating the symptoms of cancer. This approach is often considered for tumors that are localized and may not be suitable for surgical removal. There are several methods of ablation therapy, and the choice of technique depends on factors such as the size and location of the tumor, as well as the overall health of the patient. Some common types of ablation therapy include:
- Radiofrequency Ablation (RFA): This technique uses high-frequency electrical currents to generate heat, which destroys the cancerous tissue. A special needle or probe is inserted into the tumor, and the heat produced by radiofrequency waves kills the cancer cells.
- Microwave Ablation: Similar to RFA, microwave ablation uses microwaves to generate heat and destroy the cancerous cells. The procedure involves inserting a probe into the tumor, and the microwaves heat the tissue.
- Cryoablation: This method involves freezing the cancer cells. A probe is inserted into or near the tumor, and extremely cold gases or liquids are used to freeze and destroy the abnormal tissue.

Ablation therapy is often considered for patients who are not good candidates for surgery or when surgery is not feasible. It may be used to treat various types of cancers, including liver, kidney, lung, and prostate cancers, among others. However, the suitability of ablation therapy depends on the specific characteristics of the tumor and the individual patient's condition.

When conducting tumor ablation with a needle probe, various technical and adjustable factors are pivotal in determining the success of the procedure. These factors encompass power, temperature, position, safety margin, time, and other considerations specific to the chosen ablation technique. Optimizing these technical factors demands the expertise of the medical team, aiming to achieve effective tumor destruction while minimizing complications and preserving surrounding healthy tissue.

Power settings are critical in radiofrequency ablation (RFA), where the amount of energy delivered per unit of time influences the size of the ablation zone. Similarly, microwave ablation requires careful adjustment of power and frequency to ensure optimal tissue heating. Temperature control is crucial, particularly in cryoablation, where the freezing temperature of the cryoprobe dictates the extent of tissue freezing. Monitoring and regulating temperature are essential for achieving the therapeutic effect while minimizing damage to surrounding healthy tissue. In RFA, temperature monitoring ensures the targeted temperature is reached for effective ablation.

Accurate positioning of the needle probe within or near the tumor is fundamental for successful ablation. Real-time imaging guidance, such as ultrasound, CT, or MRI, aids in precise placement. Establishing a safety margin is important to account for microscopic disease and reduce the risk of local recurrence. This involves ensuring that the ablation extends beyond the visible tumor margins. The duration of energy delivery or freezing time is a critical factor. Striking a balance between achieving effective ablation and mitigating the risk of overheating or overfreezing is crucial. In cases of larger tumors, using multiple probes simultaneously or sequentially may be considered to achieve a more extensive ablation zone. For cryoablation, control over the circulation of the cryoprobe coolant helps regulate the extent and depth of tissue freezing.

Solid organ tumors often exhibit heterogeneity, which is not fully discernible through conventional imaging modalities employed in the planning of image-guided needle ablations. Needle trajectories and ablation settings, including required power and time, are typically based on tumor size and location, as observed in planning scans such as contrastenhanced CT or cone beam CT.

During needle insertion, low-resolution CT/X-ray images are taken to confirm the placement of the ablation probes. The primary objective is to accurately position the ablation energy emitter within the tumor's shaft, even in cases where tumor boundaries are not well visualized in planning or interventional scans. At times, the ablation probe position is validated using low-resolution scans after registration with planning CT scans. The current imaging techniques lack the capability to provide detailed information about the surroundings in which the ablation probe is situated. This information is crucial for the success of the ablation procedure. Registration with prior images has limitations, as the tumor may have shifted during the probe insertion process.

The blood flow (perfusion) into the tumor acts as a heat/cold sink, diminishing the effectiveness of the ablation probe. This sink effect results in a smaller zone of post-ablation necrosis than initially planned. The magnitude of this sink effect is proportional to the tumor vascularity. While microwave ablation can coagulate the vasculature and resist heat sink effects, cryo-ablation and radiofrequency ablation are susceptible to these effects, impacting the overall outcome. Advanced imaging modalities like dual-energy CT, iodine map calculations, MR perfusion, and MR permeability can provide information about tumor perfusion. However, during interventional needle ablation procedures, physicians lack live access to these advanced imaging or post-processing tools for practical reasons. Customizing the heat or cold energy used for tissue ablation based on the tumor's vascularity and the most recent probe position is essential. If physicians could obtain this information during the procedure without relying on advanced imaging tools, it would be highly beneficial.

Current needle ablation procedures face challenges related to the limited visualization of tumor boundaries during insertion, potential inaccuracies in placement due to tumor shifts, and the inability to monitor real-time tumor perfusion. Additionally, the absence of live access to advanced imaging tools hinders the customization of ablation energy based on the tumor's vascularity and the probe's current position.

The present invention provides a comprehensive solution to the challenges associated with needle ablation procedures for solid organ tumors. By introducing innovative technologies, the invention addresses the limitations of current procedures, offering real-time visualization of tumor boundaries, accurate monitoring of tumor during insertion, and live access to physiological properties of the tumor. This advancement empowers physicians to customize ablation parameter, e.g., energy, based on real-time information, significantly enhancing the precision and efficacy of needle ablation for solid organ tumors.

Independent of the grammatical term usage, individuals with male, female or other gender identities are included within the term.

The present invention relates to a method for determining an ablation parameter for an ablation treatment, comprising:
- receiving sensor data, wherein the sensor data are acquired by sensors,
   wherein the sensor data has been acquired by at least two sensors, said sensors being spatially arranged along a shaft of an ablation probe and each having a distinct sensor position, wherein the sensor data for at least one measurement parameter includes two measured values obtained at different sensor positions,
- determining the ablation parameter based on the sensor data and/or the measured values of the same measurement parameter at different sensor positions,
- outputting the ablation parameter.

The present invention pertains to a technique for establishing an ablation parameter in the context of an ablation treatment. This method can be executed through a computer, an ablation system, or a cloud computing environment. The objective of the method is to ascertain and/or furnish at least one ablation parameter. These parameters may encompass energy, position, time, safety margin, verification of needle position, verification of ablation success, and/or others. The ablation treatment targeted by this method is specifically focused on tumor ablation, with emphasis on RF (Radiofrequency), Cryo (Cryotherapy), or Thermo (Thermal) ablation methods. The present invention represents a significant advancement in the field of medical procedures, specifically concerning the ablation of tumors. The inventive method is especially designed to meticulously determine crucial ablation parameters, thereby enhancing the precision and effectiveness of ablation treatments.

The disclosed method comprises a reception of sensor data acquired by at least two sensors. The sensors are strategically positioned along the shaft of an ablation probe to capture essential information about the treatment environment. The ablation probe is for example configured as a needle and/or has a slender or elongated shape. For thermal ablation, the ablation probe typically incorporates a heating element or a thermal source at its tip and/or its distal end. This element can be activated to generate heat, which is then applied to the targeted tissue to induce coagulation or necrosis. In the case of cryoablation, the probe is equipped with a cooling mechanism, often involving the use of liquid nitrogen or argon gas. The tip and/or distal end of the ablation probe becomes extremely cold, causing the targeted tissue to freeze and ultimately leading to cell destruction. Temperature sensors are also crucial in cryoablation probes, allowing precise control of the freezing process to achieve optimal therapeutic effects. For RF ablation, the ablation probe features an electrode or an array of electrodes at its tip. When an electrical current is applied, it generates high-frequency radio waves that create localized heat. This heat is directed to the target tissue, resulting in coagulation and ablation. RF ablation probes may include impedance monitoring systems to assess tissue characteristics and ensure effective energy delivery.

The sensors in the ablation probe are preferably positioned along its length, particularly concentrated around the needle-shaped part and/or next to the distal end. This configuration ensures that the sensors are optimally situated to capture relevant data before, during and/or after the ablation procedure. The preference is to place the sensors around, before and/or after the active element, which could be the heating element for thermal ablation, the cooling part for cryoablation, or the RF emitter for RF ablation. This arrangement enhances the precision of data acquisition, as it allows for comprehensive monitoring of temperature changes and other pertinent parameters near the active element and/or distal end, providing a more accurate representation of the ablation process and/or ablation probe guidance.

The sensors along the ablation probe and/or an subsection of the ablation probe are preferably arranged at spatial intervals, with a preference for equidistant spacing. This deliberate arrangement ensures a uniform distribution along the probe's length, contributing to a comprehensive and evenly sampled data collection during the ablation procedure. The spatial positioning of these sensors becomes particularly discernible in medical imaging modalities such as CT scans. This visibility allows healthcare professionals to precisely observe and analyze the spatial relationship between the sensors and the targeted tissue, facilitating accurate monitoring and control of the ablation process. The equidistant spacing adds an element of consistency, aiding in the systematic assessment of the treatment environment and contributing to the overall efficacy of the medical intervention.

The ablation probe may comprise various sensor types, with at least two sensors of the same type arranged at spaced intervals for each type of sensor. Specifically, different types of sensors are paired, with a minimum of two sensors designed to measure the same measurement parameter. The sensor types can be configured to measure different measurement parameter, such as temperature, oxygen, glucose, or other variables. However, there are always at least two sensors dedicated to capturing the same measurement parameter.

The sensors are designed to determine a measured value for the measurement parameter at the sensor position and/or time. Since the sensors are comprised in pairs to measure the same measurement parameter, two measured values at different sensor positions are captured and provided and/or received for the same time point. Preferably, the measured values are received together with the related sensor position and/or timepoint. In other words, the sensor data comprise for at least one measurement parameter at least two measured values, wherein the at least two measured values are acquired and/or measured at different sensor positions.

The method comprises the step of determining the ablation parameter based on the sensor data and/or the measured values of the same measurement parameter at different sensor positions. In other words, at least one ablation parameter is determined, calculated, and/or generated based on at least two measurement values of the same measurement parameter, wherein the at least two measured values are related to different sensor positions. For example, the ablation parameter is determined based on the difference in the measurement value of the same parameter at different sensor positions. For instance, for a measurement parameter, the measured value is higher in a tumor or target region than in the surrounding tissue. Based on the measurement values of this parameter, the position of the tumor or target region can be identified as the ablation parameter. For example, the ablation parameter can be the relative distance of the active element or distal end and the area with the highest measurement value, e.g., the tumor. This determination may involve stating that the tumor or target region is nearer to the sensor with the higher measured value.

Determining the ablation parameter can be based on an analytical function utilizing the measured values or sensor data. Alternatively and/or additionally, determining the ablation parameter can be based on machine learning, deep learning, and/or neural networks, wherein the sensor data and/or measured values serve as inputs. For example, an analytical function may establish the ablation parameter by formulating a relationship between the measured values at different sensor positions. On the other hand, a machine learning approach could involve training a model to recognize patterns in the sensor data or measured values, enabling it to predict the ablation parameter based on these patterns.

The method comprises the step of providing and/or outputting the determined ablation parameter. The term "providing" is preferably construed as the act of making available the determined ablation parameter, either digitally or in a tangible form, to relevant stakeholders or systems involved in the medical process. This may involve transmitting the data to a user interface, a control unit, or another component of a medical device for further analysis or action. On the other hand, the term "outputting" preferable encompasses the display or presentation of the determined ablation parameter, offering a comprehensible representation of the value or information. This could involve rendering the parameter on a graphical user interface, a monitor, or any suitable output medium, providing real-time feedback to medical professionals. By clearly defining the methods of providing and outputting the ablation parameter, the patent ensures a seamless integration of this crucial information into medical workflows, ultimately improving the accuracy and efficiency of ablation procedures.

Particularly, the ablation parameter holds significant utility by serving as essential settings for an ablation device during the ablation treatment process. Preferably, the term "providing" extends to the seamless communication of the calculated ablation parameter to the ablation device, establishing a direct link between the determined parameter and the operational settings of the device. This integration ensures that the ablation treatment is tailored with precision, as the device utilizes the provided ablation parameter as configurable parameters or guidelines. These settings, derived from the ablation parameter, enable the ablation device to adapt its energy delivery, duration, or spatial targeting to suit the specific characteristics of the target region, such as a tumor. Consequently, the medical practitioner benefits from an enhanced level of customization and control over the ablation procedure, optimizing treatment efficacy and patient outcomes. This innovative feature exemplifies a sophisticated and adaptive approach to medical interventions, demonstrating a novel and valuable contribution to the field of ablation technologies.

The innovative method is characterized by the provision and determination of ablation parameters based on sensor data comprising measured values of the same measurement parameter qauried at various positions within the ablation probe. This novel approach allows for the utilization of previously undetermined or inaccurately measured variables to precisely establish the ablation parameters. In particular, this enables the determination of ablation parameters that were either overlooked or inaccurately assessed in the past. Leveraging these provided ablation parameters facilitates an improved, safer, and more accurate ablation treatment. The precise adjustment of ablation parameters based on sensor data at different positions within the ablation probe leads to optimized control of the ablation process, ultimately resulting in outstanding outcomes in medical treatment.

Particularly, the step of determining the ablation parameter comprises determining the ablation parameter based on the difference and/or comparison of the measured values of the same measurement parameter at different sensor positions. In other words, the process of determining the ablation parameter may involves evaluating the differences or making comparisons between measured values of the same measurement parameter at different sensor positions. This approach is designed to utilize variations in the measurement parameter across distinct locations, providing valuable information for establishing the ablation parameter. By focusing on the differences or comparisons, the method aims to discern specific patterns or gradients in the measurement parameter, which can be indicative of the proximity to a target region or a particular condition. For example, consider a scenario where the measured values of a temperature parameter at various sensor positions within a tissue exhibit a notable increase in a specific region. The difference or comparison of these values helps identify the temperature gradient, signaling the presence of a tumor or target region. In this case, the calculated ablation parameter could be the temperature difference between the region of interest and its surroundings, indicating the optimal settings for an ablation treatment. Another example could involve measuring electrical conductivity, oxygen concentration and/or glucose concentration within a tissue. If the conductivity values, oxygen concentration and/or glucose concentration show a substantial difference at different sensor positions, the calculated ablation parameter might be the contrast in conductivity, oxygen concentration and/or glucose concentration, guiding the ablation device to apply targeted energy to the areas with distinct conductivity, oxygen concentration and/or glucose concentration characteristics. This targeted approach improves the precision and effectiveness of the ablation treatment.

According to an embodiment of the invention, the sensor data comprise, for temperature as a measurement parameter, a first measured temperature and a second measured temperature as measured values, wherein the first temperature is related to a first temperature sensor and a first temperature sensor position, and the second temperature is related to a second temperature sensor and a second temperature sensor position, wherein the step of determining the ablation parameter comprises determining the ablation parameter based on the first and second temperatures.

In the context of temperature being the measurement parameter, the sensor data include two measured values: a first temperature obtained from a first temperature sensor located at a specific position, and a second temperature from a second temperature sensor situated elsewhere. The determination of the ablation parameter hinges on these two temperature values. For instance, consider a scenario where the temperature in a tumor is higher than in the surrounding tissue. In this case, having the temperature values allows for corrective adjustments to the position of the ablation probe. This correction is made possible by leveraging the knowledge of the sensor positions within the probe and the understanding that the temperature peaks in the tumor.

Furthermore, the knowledge of temperatures around the tumor can be utilized to calculate the required Radiofrequency (RF) heating power and/or the duration of the ablation therapy. For instance, if the temperature gradient around the tumor is known, it provides valuable information for optimizing the RF power and duration needed to effectively treat the target region. This ensures that the ablation therapy is precisely tailored to the specific temperature characteristics of both the tumor and its immediate surroundings, maximizing the efficacy of the treatment.

Temperature sensors used in ablation probes or needles come in various types, each serving a specific purpose in accurately measuring temperature during medical procedures. The ablation probe preferably comprises thermocouples as temperature sensors. Thermocouples consisting of two different metal wires generating a voltage based on temperature difference, can be embedded in ablation probes for real-time temperature monitoring. The ablation probe may comprise thermistors, with their temperature-sensitive resistors, provide varying resistance to measure temperature changes in ablation probe. The ablation probe may comprise infrared (IR) sensors, detecting emitted infrared radiation, offer a non-contact option for monitoring tissue temperature around the ablation probe. Alternatively, the ablation probe comprises fiber optic temperature sensors, utilizing changes in light properties, can be integrated into ablation needles for accurate temperature readings. Furthermore, the ablation probe may comprise resistance temperature detectors (RTDs), employing the relationship between electrical resistance and temperature, offer precise temperature indications in ablation probes. The diversity of these sensors allows for tailored temperature measurement approaches, ensuring optimal control and efficacy during ablation treatments.

According to an embodiment of the invention, the sensor data comprise, for oxygen values, e.g., an oxygen concentration, as a measurement parameter, a first measured oxygen value and a second measured oxygen value as measured values, wherein the first oxygen is related to a first oxygen sensor and a first oxygen sensor position, and the second oxygen is related to a second oxygen sensor and a second oxygen sensor position, wherein the step of determining the ablation parameter comprises determining the ablation parameter based on the first and second oxygen values.

In this embodiment of the invention, the sensor data involve oxygen concentration as the measurement parameter, incorporating two measured values: a first measured oxygen value derived from a first oxygen sensor positioned at a specific location, and a second measured oxygen value from a second oxygen sensor situated elsewhere. The determination of the ablation parameter is contingent upon these two oxygen values. For example, consider a scenario where the oxygen concentration in a tumor surpasses that in the surrounding tissue. The elevated oxygen concentration in the tumor might be attributed to increased vascularity or metabolic activity. Upon measuring the oxygen concentration, corrective adjustments to the position of the ablation probe can be made. This correction relies on understanding the sensor positions within the probe and the knowledge that the oxygen concentration peaks in the tumor. Moreover, the knowledge of oxygen concentrations around the tumor becomes valuable for calculating the required Radiofrequency (RF) heating power and/or the duration of the ablation therapy. For instance, if there is a known gradient in oxygen concentration around the tumor, this information aids in optimizing the RF power and duration, ensuring an effective and targeted ablation treatment. This tailored approach leverages the specific oxygen characteristics of both the tumor and its immediate surroundings, enhancing the precision and success of the ablation therapy.

The ablation probe may incorporate several types of oxygen sensors to accurately measure oxygen concentration during procedures. For instance, the ablation probe may include a Clark Electrode as an oxygen sensor, employing polarographic principles for electrochemical reduction of oxygen at its working electrode. Alternatively, and/or additionally, optical oxygen sensors, with luminescent materials responding to oxygen concentration changes, can be seamlessly integrated into the probe, offering non-invasive and continuous monitoring capabilities. Moreover, the ablation probe may feature amperometric sensors to measure oxygen levels, utilizing the electric current generated during the electrochemical reaction between oxygen and the working electrode. Another viable option could be fluorescence-based oxygen sensors, known for their sensitivity to oxygen concentration changes, providing accurate and reliable detection capabilities in the immediate vicinity of the probe.

According to an embodiment of the invention, the sensor data comprise, for glucose concentration as a measurement parameter, a first measured glucose value and a second measured glucose value as measured values, wherein the first glucose is related to a first glucose sensor and a first glucose sensor position, and the second glucose is related to a second glucose sensor and a second glucose sensor position, wherein the step of determining the ablation parameter comprises determining the ablation parameter based on the first and second glucose values.

In this embodiment of the invention, sensor data include, as a measurement parameter, glucose concentration with two measured values: a first measured glucose value from a first glucose sensor positioned at a specific location, and a second measured glucose value from a second glucose sensor located elsewhere. The determination of the ablation parameter involves these two glucose values. For example, if the glucose concentration in a tumor is higher than in the surrounding tissue, this could be attributed to increased metabolic activity within the tumor. Upon measuring the glucose concentration, adjustments to the position of the ablation probe can be made based on the knowledge of the sensor positions within the probe and the understanding that the glucose value is highest in the tumor. Moreover, the knowledge of glucose values around the tumor becomes valuable for calculating the required Radiofrequency (RF) heating power and/or the duration of the ablation therapy. For instance, if there is a known gradient in glucose concentration around the tumor, this information aids in optimizing the RF power and duration, ensuring an effective and targeted ablation treatment. This tailored approach leverages the specific glucose characteristics of both the tumor and its immediate surroundings, enhancing the precision and success of the ablation therapy.

The ablation probe may integrate diverse types of glucose sensors for continuous sensing during interventions. For continuous glucose monitoring, the probe could include a Continuous Glucose Monitoring (CGM) sensor, a subcutaneously implanted device that measures glucose levels in the interstitial fluid. Alternatively, an implantable fluorescence-based glucose sensor may be employed, utilizing the principle of changing fluorescence properties based on glucose concentration for real-time and continuous monitoring capabilities. Moreover, the ablation probe may incorporate a biosensor-based glucose sensor, capable of continuous monitoring by detecting glucose-specific reactions with enzymes or proteins. This biosensor approach offers real-time feedback on glucose levels. Additionally, an optical coherence tomography (OCT)-based glucose sensor could be integrated, utilizing the changes in light scattering properties to measure glucose concentrations continuously. Each of these sensors contributes to the probe's ability to continuously sense glucose levels during ablation procedures, enabling precise adjustments for optimal treatment efficacy.

In particular, the method comprises the step of determining a metabolism activity parameter, wherein the metabolism activity parameter is determined based on at least one of the measured values, wherein the ablation parameter is determined and/or verified based on the metabolism activity parameter. The metabolism activity parameter is preferably derived from measured temperature, oxygen levels, and glucose concentration. In particular, oxygen concentration and oxygen value and/or glucose concentration and glucose value are used equivalent throughout the text and/or description of the invention. In the intricate realm of tumor metabolism versus normal tissue metabolism, distinctions emerge. Tumors often exhibit heightened metabolic activity compared to surrounding healthy tissues, a phenomenon known as the Warburg effect. This elevated metabolism in tumors is associated with increased glucose consumption, altered oxygen dynamics, and temperature variations. The method outlined recognizes these disparities, utilizing the metabolism activity parameter to inform the determination and validation of ablation parameters tailored to the unique metabolic characteristics of the targeted tissue. Understanding these metabolic nuances is crucial for developing effective and precise medical interventions in the treatment of tumors.

In a preferred embodiment, the method comprises step of receiving a medical image of an object of examination. The medical image is preferably a real time image. The medical image can be configured as stream of images. The medical images can be provided by an imaging system, e.g., by a computed tomography system, by an X-ray device, a C-arm X-System, a magnetic resonance tomography system, an ultrasound system or other medical imaging systems. The medical images are for example X-Ray images, CT-images, MR-images or ultrasound images. The medical images are acquired of an object of examination, especially of a patient. The medical image comprises and/or shows a target area of the object of examination and a portion of the shaft of the ablation probe. The target area comprises a target, e.g., a tumor or object to be treated. The portion of the shaft comprised by the medical image comprises the sensors and/or shows the sensors. In other words, the medical image shows a portion of a patient, shows the target (e.g. tumor) and shows the sensors of the ablation probe. The step of providing the determined ablation parameter comprises generating and/or providing an overlay image, wherein the overlay image combines the medical image with the measured values of the relevant parameter and/or the ablation parameter. This overlay image preferably seamlessly integrates the medical image with the pertinent measured values, either of the relevant parameter or the ablation parameter. As an illustration, such overlay images might visually represent a thermal map superimposed on the medical image, depicting temperature variations within tissues. Another example could involve combining oxygen concentration data with medical images to create a composite visualization highlighting regions with distinct oxygen levels. These configurations of overlay images serve as valuable tools, providing a comprehensive and visually intuitive representation of the relationship between medical imagery and measured parameters in the context of the ablation procedure.

Preferably, the measurement parameter is related to and/or is a sodium concentration, potassium concentration, sodium-potassium-ratio, pH and/or lactate concentration. For this measurement parameter the sensor data comprise, a first measured homeostasis value and a second measured homeostasis value as measured values. The first homeostasis value is related to a first homeostasis sensor and a first homeostasis sensor position. The second homeostasis value is related to a second homeostasis sensor and a second homeostasis sensor position, wherein the step of determining the ablation parameter comprises determining the ablation parameter based on the first and second homeostasis values.

This embodiment is based on the observation, that the balance of Sodium and Potassium ions in the extracellular fluid is maintained by the active pumping of Sodium-Potassium pumps on the cell membrane. The concentration of sodium in the extracellular fluid is maintained higher and the concentration of potassium inside the cells is maintained high because of the action of these pump. These pumps are functional only until the cells are living and cell membrane is intact. In the case of Cryo-ablations, the freeze-thaw mechanism induces damage to the cell membrane disrupting the ionic equilibrium across the cell membrane. Thus, the concentration of Potassium in the extra cellular space goes up after cytolysis happens. More the concentration of Potassium in the extracellular fluid, more is the likely cytolysis in that vicinity. If the concentration of Potassium or if the Sodium/Potassium ratio can be measured at various locations within a tumor before and after Cryo-ablation, this can reveal the effectiveness of the ongoing/completed cryoablation.

Furthermore, the embodiments are reflecting that, it can be utilized to detect the presence of glucose in the extracellular space, serving as an indication of cell membrane rupture. This is crucial for identifying potential damage to cellular integrity. Secondly, the mapping enables the assessment of Na/K concentration levels before and after cell explosion, providing valuable insights into the health status of the cell membrane. Monitoring these concentrations can help in understanding membrane stability and function. Moreover, this mapping technique allows for the early detection of complications such as "Acute Lysis Syndrome," which may arise due to the sudden release of potassium ions. This early identification is especially significant during ongoing ablation procedures, potentially averting serious consequences. Additionally, the mapping facilitates the monitoring of lactate/pH accumulation, reflecting processes like coagulation, reduced perfusion, or cell death. These parameters offer a window into cellular changes occurring during ablation, aiding in treatment evaluation and adjustment. Furthermore, the ability to aspirate fluid and analyze its biochemical composition before and after ablation enhances the procedural understanding and optimization. Additionally, the possibility of performing biopsies using the aspiration needle shaft itself presents a streamlined approach to tissue sampling, improving procedural efficiency and accuracy.

The method preferably comprises a step of determining a measured value distribution and/or interpolation of the measured values of the measurement parameter measured by sensors at different sensor positions, wherein the step of providing the determined ablation parameter, comprises generating and/or providing the overlay image, wherein the overlay image combines the medical image with the measured value distribution and/or the measured value interpolation.

Preferably, the method comprises a step of receiving a medical image of an object of examination, e.g., as described before. The medical image includes a target area of the object of examination and a portion of the shaft of the ablation probe, wherein the target area is characterized by a different average value of the measurement parameter than the surroundings of the target area. The target area is characterized by the different average value means especially, that in general or as prior knowledge, the target area and/or tissue of the target area has by nature a different average value of the measurement value. For example, the target or target area is a tumor, wherein a tumor has by nature a higher temperature, oxygen value, glucose concentration and/or metabolism than normal tissue. The step of determining the ablation parameter comprises determining as ablation parameter a positioning check information of the ablation probe based on the measured values of the measurement parameter. The position check is based on the idea, that by measuring the measurement parameter at different positions along the shaft, the position of the target relative to the ablation probe can be determined based on the measured values. For example, by using rules and/or knowledge, that the target or tumor must be nearer to the sensor position with the higher temperature, oxygen concentration, glucose concentration or metabolism than to the sensor position with the lower measured values.

Furthermore, the step of determining the ablation can comprise determining as ablation parameter a safety margin for the ablation based on the measured values of the measurement parameter. This can be based on the knowledge of the distribution of the measured values, e.g., by estimating or determining the metabolism, diffusion or blood circulation based on the measured values, e.g., for a target with high diffusion and/or blood circulation it is expected a high heat sink effect, wherein a higher safety margin can be useful. For example, based on the measured values a grade of the tumor is determined, wherein based on the grade, the safety margin is determined.

Furthermore, the step of determining the ablation parameter preferably comprises determining as ablation parameter a residual check information of the ablation based on the measured values of the measurement parameter. Based on the measured values, e.g., the temperature, oxygen or glucose concentration the success and/or efficiency of the ablation treatment can be determined. When the tumor or target was treated completely successful, the measured values of the measurement parameter after the ablation treatment should be similar to the surrounding and/or normal tissue. In case that the ablation was not completely successful, the measured values, e.g., temperature, oxygen and/or glucose concentrations, should differ to the one of the normal tissues, since active tumor tissue is remaining.

In an optional embodiment it can be provided that instead of spatially separated sensors along the ablation probe, the ablation probe only includes one such sensor for measuring the measured values of the measurement parameter. The first measured value is recorded at a different time from the second measured value. The ablation parameter to be determined can then refer to a time-changing ablation value or characteristic. This suggests that the design of the ablation probe might include just one sensor, as opposed to multiple sensors spaced along the length of the probe. This single sensor would take measurements at different times, with the first and second measurements being taken at different points in time. The parameter to be determined from these measurements could be a value that changes over time.

In another optional embodiment of the invention, it is envisaged that the ablation probe, especially the shaft, is variably designed, for example, it can assume two different functional and/or constructive states, between which can be switched. For instance, the ablation probe can assume a treatment state and a measurement state. In the treatment state, the ablation probe is equipped with the active element for ablation, e.g., RF generator, while in the measurement state, the ablation probe is equipped with sensors for measuring the 'measured values'. For this purpose, the ablation probe can be hollow, especially designed as a hollow needle, into which the sensors and/or the active element can be inserted depending on the state.

Another optional embodiment of the invention provides that the sensor(s) along the ablation probe, especially along the shaft, can be positioned, movable, and/or displaceable. For example, based on imaging, the sensors can be positioned so that they are close to a sensitive and/or to be supported area, such as the spine, and it can be ensured that this is not damaged in the ablation treatment.

This suggests that the ablation probe can switch between two states: a treatment state, where it is equipped with an active element for ablation (like an RF generator), and a measurement state, where it is equipped with sensors for taking measurements. The probe could be designed as a hollow needle, allowing the sensors or active element to be inserted as needed. Additionally, the sensors could be movable or adjustable along the probe, allowing them to be positioned close to sensitive areas (like the spine) to ensure these areas are not damaged during the ablation procedure.

A further object of the invention is a tumor ablation device comprising:
- an ablation probe, said ablation probe comprising a treatment section for cryo, thermal, heat, or RF treatment of the tumor, wherein said ablation probe is positionable or insertable into a patient to be treated,
- characterized in that the ablation probe comprises at least two sensors, wherein the at least two sensors are configured to acquire sensor data, said sensors being spatially arranged along a shaft of the ablation probe, each sensor having a distinct sensor position, wherein the sensor data for at least one measurement parameter includes two measured values obtained at different sensor positions.

The tumor ablation device, especially the ablation probe, is configured to treat a tumor in a patient in a cryo, thermal, heat and/or RF ablation treatment. The tumor is also called target and/or the region with the tumor in the patient may be also named as target area. The ablation probe can be configured as an ablation needle. The ablation probe comprises a distal and a proximal end. The distal end is the end of the probe that is farthest away from the point of origin or closest to the target tissue within the patient's body. It is the end that comes into direct contact with the tissue to be treated or ablated. The proximal end is the end of the probe that is closer to the point of origin or located outside the patient's body. This end is for example connected to a control unit, an interface module and/or monitoring equipment used by the healthcare professional performing the procedure. The ablation probe is configured that the distal end can be inserted in the patient's body. The treatment section is preferably located at the distal end and/or in the region of the distal end. The treatment section preferably comprises the active element and/or active area.

For cryoablation, the treatment section of the probe is typically a cryoprobe that contains a cooling agent, such as liquid nitrogen or argon gas. The cooling agent is used to freeze and destroy targeted tissue. For thermal ablation, the treatment section of the probe generates heat to ablate (destroy) tissue. This can be achieved using different techniques such as laser ablation, microwave ablation, or high-intensity focused ultrasound (HIFU). The specific component producing the heat varies based on the method employed. RF ablation uses high-frequency electrical currents to generate heat and ablate tissue. The treatment section of an RF ablation probe comprises an electrode that delivers RF energy to the targeted tissue.

The ablation probe, e.g., the ablation needle, comprises several sensors. The sensors are preferably located between the distal and the proximal end. Particularly, the sensors are arranged and/or located next and/or nearer to the distal end of the ablation probe. The ablation probe has an elongated structure, wherein the elongation is defined from the distal to the proximal end of the ablation probe. The sensors are spatially arranged along the longitudinal extension. The distance between the sensors is preferably at least one millimeter and/or less than five millimeters. The probe comprises at least two sensors spatially arranged and configured to measure the same measurement parameter. For example, the probe comprises at least two temperature sensors spatially arranged along the shaft and/or the probe comprises at least two oxygen sensors spatially arranged along the shaft and/or the probe comprises at least two glucose sensors spatially arranged along the shaft. Each sensor having a distinct sensor position, wherein the sensor data for at least one measurement parameter includes two measured values obtained at different sensor positions.

The tumor ablation device, for example the ablation probe, comprises an interface module. The interface module is connected for data exchange with the sensors of the ablation probe. The interface module is configured to provide the sensor data to a method for determining an ablation parameter and/or to provide it to an internal or external user or device. The interface module can be configured to provide the sensor data wireless and/or wired.

Preferably, the tumor ablation device according comprises a control unit. The control unit can be configured as an interface for a user and/or configured as a computing unit. The control unit is preferably connected for data exchange with the ablation probe and/or the interface unit. The control unit is configured to receive a provided ablation parameter and to control and/or to operate the ablation probe accordingly. Optionally, the control unit is configured to execute the method to determine the ablation parameter.

A further object of the invention is a computer program product comprising a non-transitory computer-readable medium storing instructions that, when executed by a processor, cause the processor to perform the steps of the method for determining the ablation parameter.

Additional benefits, effects, and embodiments arise from the attached figures and their description, illustrating:
- Figure 1: a tumor ablation device comprising an ablation probe;
- Figure 2: an ablation probe of the tumor ablation device;
- Figure 3: data exchange of the tumor ablation device;
- Figure 4: a flow chart of a method for determining an ablation parameter;
- Figures 5a, b: an overlay image.

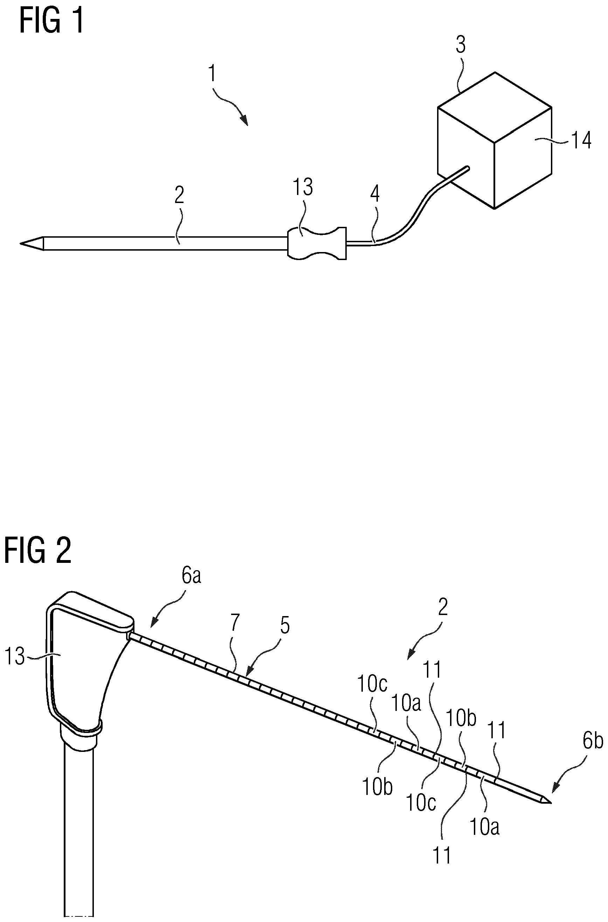

Referring to Fig. 1 a tumor ablation device 1 comprising an ablation probe 2 constructed in accordance with one embodiment of the invention, will now be described. The tumor ablation device 1 generally comprises an ablation probe 2 configured for introduction into the body of a patient for ablative treatment of target tissue, a source of ablation energy, and in particular a radio frequency (RF) generator 3, and a cable 4 electrically connecting the ablation probe 2 to the RF generator 3. The source of ablation energy can alternatively be a heating element or a cooling element or cooling system with nitrogen, e.g., for cryoablation.

Referring now to Fig. 2, the ablation probe 2 will be described in further detail. The ablation probe 2 comprises an elongated, shaft 5 having a proximal end 6a and a distal end 6b. The probe shaft 5 is composed of an electrically conductive material, such as stainless steel. The probe shaft 5 has a suitable length, typically in the range from 5 cm to 30 cm, preferably from 10 cm to 25 cm, and an outer diameter consistent with its intended use, typically being from 0.7 mm to 5 mm, usually from 1 mm to 4 mm.

The ablation probe 2 further may comprise a sheath 7 disposed on the probe shaft 5. In the illustrated embodiment, the sheath 7 is affixed to the probe shaft 5 and may be applied to the probe shaft 5 using any suitable means. For example, the insulative sheath 7 can be applied to the probe shaft 5 as a heat shrink or can be extruded onto the probe shaft 5. The sheath 7 is composed of an electrically insulative material, such as Fluorinated Ethylene Propylene (FEP), and extends the entire length of the probe shaft, with the exception of a distal tip of the probe shaft 5 In this manner, an RF ablation electrode 9 is formed by the exposed portion of the distal tip.

The shaft 5 is visible in X-ray images, CT images, MR images and/or ultrasound images. The ablation probe 2 comprises several sensors 10. The sensors 10 are positioned along the shaft 5, especially in an area of the distal end. Each sensor has a sensor position 11, wherein the sensors 10 and the sensor positions 11 are displaced along the shaft 5. The sensors 10 and/or the sensor positions are visible in in X-ray images, CT images, MR images and/or ultrasound images.

The sensors 10 are configured to measure a value of measurement parameter. The ablation probe 2 can comprise different types of sensors 10, e.g., temperature sensors 10a, oxygen sensors 10b and/or glucose sensors 10c. Each sensor type used in the probe, especially for the method according to the invention, is comprised at least twice. E.g., the ablation probe 2 comprises at least two displaced temperature sensors 10a, at least two displaced oxygen sensors 10b and/or at least two displaced glucose sensors 10c. The sensors 10 are configured to acquire sensor data, wherein the sensor data comprise a measured value of a measurement parameter.

The tumor ablation device 1 comprises an interface module 13. The interface module 13 is connected to the sensors 10 and configured to provide the sensor data to a method for determining an ablation parameter and/or to provide it to an internal or external user or device. The tumor ablation device 1 further comprises a control unit 14, wherein the control unit 14 is configured to receive a provided ablation parameter and to control and/or to operate the ablation probe 2 accordingly.

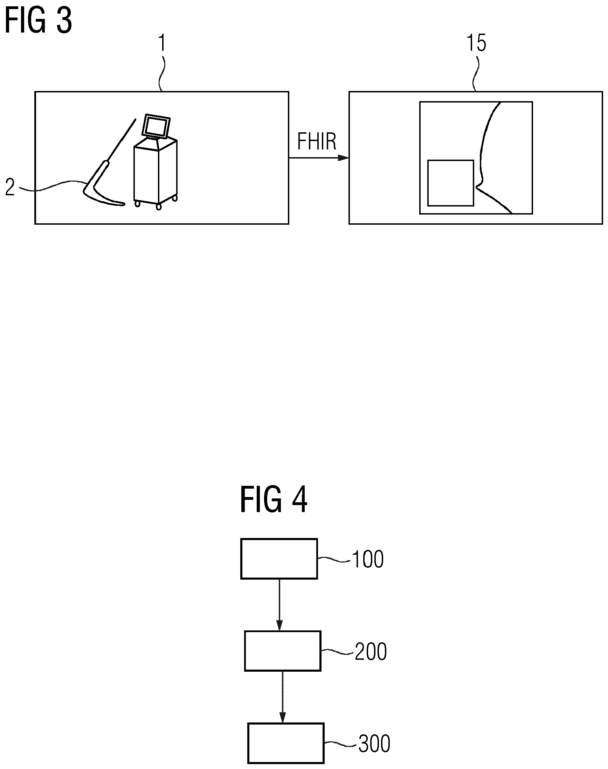

Figure 3 depicts a data exchange scenario involving a tumor ablation device 1 and a monitoring system 15 dedicated to overseeing the ablation probe 2 and/or the ablation process. This monitoring system 15 relies on medical imaging data, particularly X-ray images, to facilitate its functions. Primarily, the monitoring system 15 is tailored for needle guidance, ensuring precise navigation of the ablation probe 2 towards the target tumor during medical imaging procedures.

Integral to this setup is the connection between the monitoring system 15 and the ablation device 2, notably interfacing with the interface unit 13. Sensor data from the ablation device 1 are relayed to the monitoring system 15, enabling real-time feedback and analysis. Moreover, the monitoring system 15 is configured to execute the innovative method for determining optimal ablation probe parameters as per the invention.

Once the parameters are determined, the monitoring system 15 seamlessly communicates them back to the tumor ablation device 1, enhancing its efficacy and accuracy during the ablation procedure. This data exchange mechanism is facilitated through the Fast Healthcare Interoperability Resources (FHIR) device, ensuring efficient and standardized communication protocols within the medical environment.

Figure 4 illustrates a flowchart for an embodiment of a method for determining an ablation parameter. The method consists of three main steps: receiving (Step 100), determining (Step 200), and providing (Step 300).

The heat-generating capacity of tumors is a dynamic variable, which changes throughout their progression. Two key parameters work together to determine this capacity: tumor cell metabolism, generating heat as a byproduct, and vascularization within and around the tumor, which supplies the oxygen and nutrients required for tumor cell proliferation. In the early stages of tumor development, a lack of vasculature limits tumor cell metabolism, tumor growth, and, ultimately, heat generation.

During the receiving step 100, sensor data acquired by at least two sensors 10 are collected. These sensors are spatially arranged along a shaft 5 of an ablation probe 2.

Each sensor 10 possesses a distinct sensor position. The sensor data includes measurements for at least one parameter, with each parameter comprising two measured values obtained from different sensor positions.

The ablation probe 2 is fitted with thermocouples (temperature measuring sensors) at various lengths along the shaft of the probe. 2 These thermocouples can measure real-time temperature and transmit this data to ablation guide software. While the illustration below depicts three thermocouples, in practice, more thermocouples could be used.

In the determining step 200, the ablation parameter is computed based on the sensor data and/or the measured values of the same parameter at different sensor positions. As ablation parameter can comprise a tumor margin, a position of the ablation probes relative to a tumor, a grade of the tumor, a distribution of normal and tumor tissue around the probe, an ablation power, temperature and/or an overlay image. In particular, the ablation parameter can be a position of the tumor relative to the sensors and/or a distribution of the measurement parameter around the ablation probe and/or relative to the sensors.

Finally, in step 300, the determined ablation parameter is provided. This provision may include visual presentation and/or utilization for controlling the ablation probe 2.

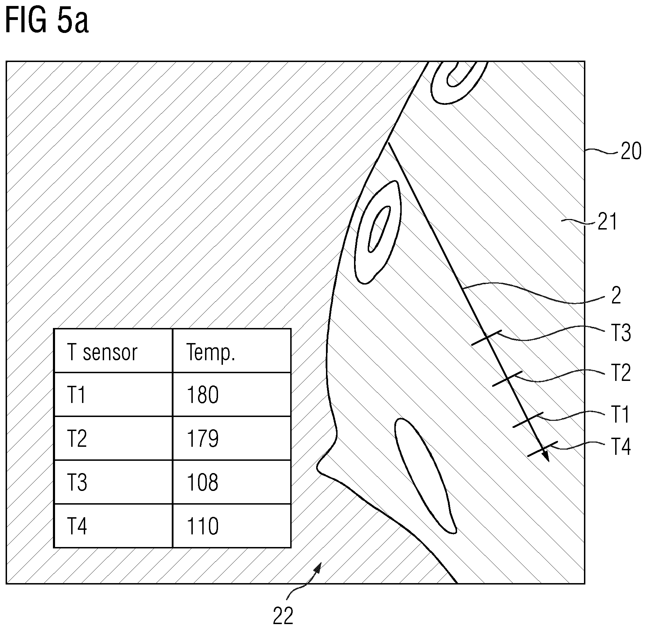

Figure 5a depicts an overlay image 20 based on a medical image 21, e.g., an X-ray of a patient. The overlay image 20 reveals a tumor and its surrounding area, along with an ablation probe 2. The ablation probe 2 is essentially positioned within the tumor, particularly with its distal end housing the RF emitter. Adjacent to the ablation probe 2 are the sensor positions T1, T2, T3, T4 marked on the overlay. In this setup, the probe accommodates four temperature sensors at T1, T2, T3, and T4. The overlay image 20 also includes a temperature display 22 representing the temperatures 23 measured by these sensor positions T1, T2, T3, T4.

It's evident from the display that T1 and T2 exhibit significantly higher temperatures compared to T3 and T4. This suggests that the ablation probe 2, along with the sensors for T1 and T2, is either within the tumor or closest to it. Conversely, the positions of sensors T3 and T4 correspond to temperatures indicating they are outside the tumor. The overlay image 20 serves as an ablation parameter, providing users with visual feedback on whether the ablation probe 2 is correctly positioned or if an adjustment in position is necessary.

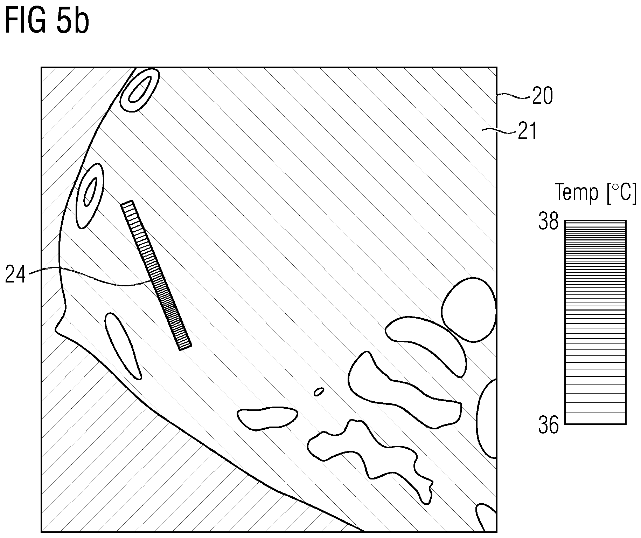

Figure 5b presents an overlay image 20 that essentially corresponds to that of Figure 5a. However, in contrast, temperatures are not individually specified for positions and not tabulated here. Instead, based on the temperatures from T1, T2, T3, and T4, an interpolation of the temperature has been determined, and the temperature distribution 24 along and/or around the ablation probe 2 is depicted as a heatmap. Through the heatmap, the user can discern the position of the tumor, as it is presumed to be in the area of highest temperature. The overlay image 20 serves here as an ablation parameter displayed to the user.

## Claims

1. A method for determining an ablation parameter for an ablation treatment, comprising:
- Receiving (100) sensor data acquired by at least two sensors (10), said sensors (10) being spatially arranged along a shaft (5) of an ablation probe (2), each sensor having a distinct sensor position (11, T1, T2, T3, T4), wherein the sensor data for at least one measurement parameter includes two measured values obtained at different sensor positions (11, T1, T2, T3, T4);
- Determining (200) the ablation parameter based on the sensor data and/or the measured values of the same measurement parameter at different sensor positions (11, T1, T2, T3, T4);
- Providing (300) the determined ablation parameter.

2. A method according to claim 1, wherein the step of determining (200) the ablation parameter comprises determining the ablation parameter based on the difference and/or comparison of the measured values of the same measurement parameter at different sensor positions (11, T1, T2, T3, T4) .

3. A method according to claims 1 or 2, wherein the sensor data comprise, for temperature as a measurement parameter, a first measured temperature and a second measured temperature as measured values, wherein the first temperature is related to a first temperature sensor and a first temperature sensor position (11, T1), and the second temperature is related to a second temperature sensor and a second temperature sensor position (11, T2), wherein the step of determining the ablation parameter comprises determining the ablation parameter based on the first and second temperatures.

4. A method according to one of the preceding claims, wherein the sensor data comprise, for oxygen concentration as a measurement parameter, a first measured oxygen value and a second measured oxygen value as measured values, wherein the first oxygen value is related to a first oxygen sensor and a first oxygen sensor position (11), and the second oxygen value is related to a second oxygen sensor and a second oxygen sensor position (11), wherein the step of determining the ablation parameter comprises determining the ablation parameter based on the first and second oxygen values.

5. A method according to one of the preceding claims, wherein the sensor data comprise, for sodium concentration, potassium concentration, sodium-potassium-ratio, pH and/or lactate concentration as a measurement parameter, a first measured homeostasis value and a second measured homeostasis value as measured values, wherein the first homeostasis value is related to a first homeostasis sensor and a first homeostasis sensor position (11), and the second homeostasis value is related to a second homeostasis sensor and a second homeostasis sensor position (11), wherein the step of determining the ablation parameter comprises determining the ablation parameter based on the first and second homeostasis values.

6. A method according to one of the preceding claims, wherein the sensor data comprise, for glucose concentration as a measurement parameter, a first measured glucose value and a second measured glucose value as measured values, wherein the first glucose is related to a first glucose sensor and a first glucose sensor position (11), and the second glucose is related to a second glucose sensor and a second glucose sensor position (11), wherein the step of determining the ablation parameter comprises determining the ablation parameter based on the first and second glucose values.

7. A method according to one of the preceding claims, comprising the step of determining a metabolism activity parameter, wherein the metabolism activity parameter is determined based on at least one of the measured values, wherein the ablation parameter is determined and/or verified based on the metabolism activity parameter.

8. A method according to one of the preceding claims, comprising a step of receiving a medical image (21) of an object of examination, wherein the medical image (21) includes a target area of the object of examination and a portion of the shaft (5) of the ablation probe (2), wherein the portion comprises the sensors (10), wherein the step of providing (300) the determined ablation parameter comprises generating and/or providing an overlay image (20), wherein the overlay image (20) combines the medical image (21) with the measured values of the relevant parameter and/or the ablation parameter.

9. A method according to claim 8, comprising a step of determining a measured value distribution (23) and/or interpolation of the measured values of the measurement parameter measured by sensors (10) at different sensor positions (11, T1, T2, T3, T4), wherein the step of providing (300) the determined ablation parameter, comprises generating and/or providing the overlay image (20), wherein the overlay image (20) combines the medical image (21) with the measured value distribution (23) and/or the measured value interpolation.

10. A method according to one of the preceding claims, comprising a step of receiving a medical image (21) of an object of examination, wherein the medical image (21) includes a target area of the object of examination and a portion of the shaft of the ablation probe (2), wherein the target area is **characterized by** a different average value of the measurement parameter than the surroundings of the target area, wherein the step of determining (200) the ablation parameter comprises determining as ablation parameter a positioning check information of the ablation probe (2) based on the measured values of the measurement parameter.

11. A method according to one of the preceding claims, comprising a step of receiving a medical image (21) of an object of examination, wherein the medical image (21) includes a target area of the object of examination and a portion of the shaft (5) of the ablation probe (2), wherein the target area is **characterized by** a different average value of the measurement parameter than the surroundings of the target area, wherein the step of determining the ablation parameter comprises determining as ablation parameter a safety margin for the ablation based on the measured values of the measurement parameter.

12. A method according to one of the preceding claims, comprising a step of receiving a medical image (21) of an object of examination, wherein the medical image includes a target area of the object of examination and a portion of the shaft (5) of the ablation probe, wherein the target area is **characterized by** a different average value of the measurement parameter than the surroundings of the target area, wherein the step of determining the ablation parameter comprises determining as ablation parameter a residual check information of the ablation based on the measured values of the measurement parameter.

13. A tumor ablation device (1) comprising an ablation probe (2) and an interface module (12), wherein said ablation probe (2) comprises a treatment section for cryo, thermal, heat, or RF treatment of a tumor, wherein said ablation probe is positionable or insertable into a patient to be treated, **characterized in that** the ablation probe (2) comprises at least two sensors (10), wherein the at least two sensors (10) are configured to acquire sensor data, said sensors being spatially arranged along a shaft (5) of the ablation probe (2), each sensor (10) having a distinct sensor position (11, T1, T2, T3, T4), wherein the sensor data for at least one measurement parameter includes two measured values obtained at different sensor positions, wherein the interface module (12) is configured to provide the sensor data to a method for determining a an ablation parameter and/or to provide it to an internal or external user or device.

14. A tumor ablation device (1) according to claim 13, further comprising a control unit (14), wherein the control unit (14) is configured to receive a provided ablation parameter and to control and/or to operate the ablation probe (2) accordingly.

15. A computer program product comprising a non-transitory computer-readable medium storing instructions that, when executed by a processor, cause the processor to perform the steps of the method according to any one of claims 1 to 12.
